# EUROPÄISCHE PATENTANMELDUNG

(11) **EP 1 006 103 A1**
(43) Veröffentlichungstag der Anmeldung: **07.06.2000**
(21) Anmeldenummer: 99123454.3
(22) Anmeldetag: 25.11.1999
(51) Int. Cl.: C07C 213/06, C07C 219/06

(54) **Verfahren zur Herstellung von niedrigviskosen Esterquatzubereitungen**

(30) Priorität: 04.12.1998 DE 19855954
(71) Anmelder: Cognis Deutschland GmbH, 40589 Düsseldorf (DE)
(72) Erfinder: Bigorra Llosas, Joaquin, Dr., 08203 Sabadell (ES); Bonastre Nuria, Gilabert, Dr., 08210 Barbera del Vall (ES); Pi Subirana, Rafael, Dr., 08400 Granollers (ES)

(57) **Zusammenfassung**

Vorgeschlagen wird ein Verfahren zur Herstellung von niedrigviskosen Esterquatzubereitungen durch Veresterung bzw. Umesterung von Gemischen aus Fettsäuren und Triglyceriden mit Triethanolamin und nachfolgende Quaternierung der resultierenden Ester mit Dimethylsulfat in alkoholischer Lösung, bei dem man
(a) Mischungen von Talgfettsäure und gehärtetem Rindertalg - bezogen auf die Fettsäurekomponenten - im molaren Verhältnis 85 : 15 bis 95 : 5 einsetzt und
(b) ein molares Einsatzverhältnis Fettsäurekomponente : Triethanolamin im Bereich von 2 : 1 bis 2,1 : 1 einstellt.

## Beschreibung

### Gebiet der Erfindung

Die Erfindung befindet sich auf dem Gebiet der kationischen Tenside und betrifft ein neues Verfahren, das die Herstellung von niedrigviskosen Esterquatzubereitungen ermöglicht.

### Stand der Technik

Unter der Bezeichnung 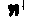 Esterquats" werden im allgemeinen quaternierte Fettsäuretriethanolaminester-salze verstanden. Es handelt sich dabei um bekannte Stoffe, die man nach den einschlägigen Methoden der präparativen organischen Chemie erhalten kann. In diesem Zusammenhang sei auf die Internationale Patentanmeldung **WO 91/01295** (Henkel) verwiesen, nach der man Triethanolamin in Gegenwart von unterphosphoriger Säure mit Fettsäuren partiell verestert, Luft durchleitet und anschließend mit Dimethylsulfat oder Ethylenoxid quaterniert. Aus der Deutschen Patentschrift **DE 4308794 C1**(Henkel) ist überdies ein Verfahren zur Herstellung fester Esterquats bekannt, bei dem man die Quaternierung von Triethanolaminestern in Gegenwart von geeigneten Dispergatoren, vor-zugsweise Fettalkoholen, durchführt. Übersichten zu diesem Thema sind beispielsweise von R.Puchta et al. in **Tens.Surf.Det., 30, 186 (1993),** M.Brock in **Tens.Surf.Det 30, 394 (1993),** R.Lagerman et al. in **J.Am.Oil.Chem.Soc., 71, 97 (1994)** sowie I.Shapiro in **Cosm.Toil. 109, 77 (1994)** erschienen. Für die Herstellung von Wäscheweichspülmitteln haben sich insbesondere Esterquats auf Basis von technischer C₁₆₋C₁₈₋Talgfettsäure bzw. Rindertalg als geeignet erwiesen. Dabei besteht jedoch das Problem, daß zwar sowohl die verdünnten Anwendungslösungen, als auch die bei der großtechnischen Herstellung anfallenden Konzentrate eine hinreichend niedrige Viskosität aufweisen, letztere jedoch beim Umpumpen und allen anderen Prozessen, in denen sie Scherkräften unterworfen werden, spontan vergelen und dann nur noch mit großen Schwierigkeiten zu handhaben sind. In der Praxis versucht man der Gelbildung zu begegnen, indem man den Konzentraten Amidoamine zusetzt, was jedoch sowohl aus anwendungstechnischer als auch ökonomischer Sicht allenfalls als Notlösung angesehen werden kann.

Demzufolge hat die Aufgabe der vorliegenden Erfindung darin bestanden, unter Verzicht auf Viskositätsregulatoren ein verbessertes Verfahren zur Herstellung von Esterquatzubereitungen auf Basis von Talgfettsäure und Rindertalg zur Verfügung zu stellen, bei dem sowohl die verdünnten als auch konzentrierten Lösungen eine hinreichend niedrige Viskosität aufweisen, und die Konzentrate unter Einwirkung von Scherkräften nicht vergelen.

### Beschreibung der Erfindung

Gegenstand der Erfindung ist ein Verfahren zur Herstellung von niedrigviskosen Esterquatzubereitungen durch Veresterung bzw. Umesterung von Gemischen aus Fettsäuren und Triglyceriden mit Triethanolamin und nachfolgende Quaternierung der resultierenden Ester mit Dimethylsulfat in alkoholischer Lösung, bei dem man
(a) Mischungen von Talgfettsäure und gehärtetem Rindertalg - bezogen auf die Fettsäurekomponenten - im molaren Verhältnis 85: 15 bis 95 : 5 einsetzt und
(b) ein molares Einsatzverhältnis Fettsäurekomponente : Triethanolamin im Bereich von 2 : 1 bis 2,1 : 1 einstellt.

Überraschenderweise wurde gefunden, daß konzentrierte Zubereitungen von Esterquats auf Basis von Talgfettsäure und Rindertalg dann bei Scherung nicht vergelen, sondern niedrigviskos bleiben, wenn man innerhalb enger Grenzen zum einen ein definiertes molares Verhältnis zwischen Säure und Triglycerid und zum anderen ein weiteres definiertes molares Verhältnis zwischen Fettsäure und Alkanolamin einstellt.

### Veresterung/Umesterung

Die Veresterung bzw. Umesterung der Mischungen aus Triglyceriden, Fettsäuren und Alkanolaminen, die gegebenenfalls weiterhin noch Polyole enthalten können, kann in an sich bekannter Weise, d.h. bei Temperaturen im Bereich von 180 bis 220 und vorzugsweise 190 bis 200°C durchgeführt werden; ein solches Verfahren wird beispielsweise in der Deutschen Patentschrift **DE 19611999 C2** (Henkel) beschrieben. Es empfiehlt sich, das Kondensationswasser kontinuierlich aus dem Reaktionsansatz zu entfernen, um so das Gleichgewicht auf die Seite der Zielprodukte zu verlagern. Als Katalysatoren kommen Hypophosphorsäure und deren Alkali- und/oder Erdalkalisalze in Betracht, die in Mengen von 0,01 bis 0,5 Gew.-% - bezogen auf die Einsatzstoffe - eingesetzt werden können. Besonders vorteilhaft ist die Mitverwendung von Reduktionsmitteln, wie insbesondere Natriumborhydrid, in Mengen von 10 bis 50 Gew.-% - bezogen auf den Katalysator - da die Co-Katalysatoren nicht nur die Umesterung unterstützen, sondern auch die Farbqualität der Reaktionsprodukte verbessern. Nichtumgeesterte Triglyceride und nichtabreagierte freie Fettsäuren verbleiben in der Mischung.

### Quaternierung

Auch die Quaternierung kann in an sich bekannter Weise erfolgen. Die Umsetzung mit Dimethylsulfat findet typischerweise in alkoholischer Lösung statt, wobei die Verwendung von Isopropylalkohol bevorzugt ist. In der Regel wird die Quaternierung bei Temperaturen im Bereich von 50 bis 90 und vorzugsweise 60 bis 70°C durchgeführt, wobei sich die Menge an Alkylierungsmittel danach richtet, welches Eigenschaftsprofil eingestellt werden soll. Demzufolge kann die Einsatzmenge zwischen 50 und 105 und vorzugsweise 90 bis 100 Mol-% - bezogen auf den für die Quaternierung zur Verfügung stehenden Stickstoff - liegen. Falls erforderlich, kann nichtumgesetztes Alkylierungsmittel beispielsweise durch Zugabe von Monoethanolamin oder Glycin nachträglich zerstört werden.

### Gewerbliche Anwendbarkeit

Die nach dem erfindungsgemäßen Verfahren erhältlichen Esterquatzubereitungen eignen sich insbesondere zur Herstellung von Wäschweichspülmitteln, in denen sie in Mengen von 1 bis 50, vorzugsweise 3 bis 30 und insbesondere 5 bis 15 Gew.-% - bezogen auf die Mittel - enthalten sein können.

### Beispiele

**Allgemeine Herstellvorschrift.** In einem 1-l-Dreihalskolben mit Rührer, Tropftrichter und Destillationsaufsatz wurden bei 70°C Talgfettsäure, gehärteter Rindertalg und 0,65 g Hypophosphorsäure vermischt. Anschließend wurde der Druck bis auf 35 mbar vermindert, portionsweise Triethanolamin zugegeben und schließlich die Temperatur bis auf 165°C gesteigert. Nach Beendigung der Zugabe wurde das Vakuum auf 2 mbar abgesenkt und die Mischung 3 Stunden gerührt, bis kein Wasser mehr abgeschieden wurde. 0,9 mol des resultierenden Talgfettsäuretriethanolaminesters wurden bei 60°C in 78 g Isopropylalkohol gelöst. Anschließend wurde die Mischung portionsweise mit 0,86 mol Dimethylsulfat versetzt und 5 h gerührt. Es wurde eine gelblich gefärbte Paste erhalten. Die Einsatzkonzentrationen und die Viskositäten sind in Tabelle 1 zusammengefaßt. Bestimmt wurden die Viskositäten (Brookfleld RVT-Viskosimeter, 20°C, 10 Upm, Spindel 1) 5 Gew.-%iger wäßriger Verdünnungen sowie von Konzentraten mit 20 Gew.-% Aktivsubstanzgehalt vor und nach Scherung (5 min bei 13.500 Upm). Beispiel 1 ist erfindungsgemäß, die Beispiele V1 bis V4 dienen zum Vergleich.

**Tabelle 1**

| **Einsatzkonzentrationen und Viskositäten** | | | | | |
|---|---|---|---|---|---|
| **Esterquats** | **1** | **V1** | **V2** | **V3** | **V4** |
| Molares Verhältnis Talgfettsäure : Rindertalg* | 90 : 10 | 50 : 50 | 80 : 20 | 80 : 20 | 90:10 |
| Molares Verhältnis Fettsäure : Triethanolamin | 2,1 : 1 | 2,5 : 1 | 1,9 : 1 | 2,1 : 1 | 1,9 : 1 |

| **Viskosität *[mPas]*** | | | | | |
|---|---|---|---|---|---|
| - Verdünnung (5 Gew.-% Aktivsubstanz) | 106 | 520 | 186 | 314 | 92 |
| - Konzentrat (20 Gew.-% Aktivsubstanz) | 46 | 60 | 82 | 98 | 54 |
| - Konzentrat nach Scherung | 250 | 2560 | 1780 | 5350 | 1360 |

| | | | | | |
|---|---|---|---|---|---|
| *) Molare Angaben bezogen auf Fettsäuregruppen | | | | | |

## Patentansprüche

1. Verfahren zur Herstellung von niedrigviskosen Esterquatzubereitungen durch Veresterung bzw. Umesterung von Gemischen aus Fettsäuren und Triglyceriden mit Triethanolamin und nachfolgende Quaternierung der resultierenden Ester mit Dimethylsulfat in alkoholischer Lösung, **dadurch gekennzeichnet**, daß man
(a) Mischungen von Talgfettsäure und gehärtetem Rindertalg - bezogen auf die Fettsäurekomponenten - im molaren Verhältnis 85 : 15 bis 95 : 5 einsetzt und
(b) ein molares Einsatzverhältnis Fettsäurekomponente : Triethanolamin im Bereich von 2 : 1 bis 2,1 : 1 einstellt.

2. Verfahren nach Anspruch 1, **dadurch gekennzeichnet,** daß man den Esterquats keine Amidoamine als Viskositätsregulatoren zusetzt.
